(19) Europäisches Patentamt
European Patent Office
Office européen des brevets

(11) Veröffentlichungsnummer : **0 287 882 B1**

(12) # EUROPÄISCHE PATENTSCHRIFT

(45) Veröffentlichungstag der Patentschrift : **02.05.91 Patentblatt 91/18**

(51) Int. Cl.$^5$ : **C07C 217/54**, C07K 1/04, C07C 59/68

(21) Anmeldenummer : **88105352.4**

(22) Anmeldetag : **02.04.88**

(54) **Synthese von Peptidamiden mittels Festphasenmethode unter Verwendung von säurelabilen Ankergruppen.**

(30) Priorität : **08.04.87 DE 3711866**

(43) Veröffentlichungstag der Anmeldung : **26.10.88 Patentblatt 88/43**

(45) Bekanntmachung des Hinweises auf die Patenterteilung : **02.05.91 Patentblatt 91/18**

(84) Benannte Vertragsstaaten : **AT BE CH DE ES FR GB GR IT LI LU NL SE**

(56) Entgegenhaltungen :
**EP-A- 0 021 324**
**FR-A- 2 428 629**
**GB-A- 1 539 897**
**GB-A- 2 028 820**
**GB-A- 2 169 901**
**US-A- 2 683 737**

(56) Entgegenhaltungen :
**US-A- 4 101 721**
**TETRAHEDRON LETTERS, Band 22, Nr. 28, 1981; JAMES P. TAM et al.: "Design and synthesis of a multi-detachable benzhydrylamino-resin for solid phase peptide synthesis", Seiten 2851-2854**

(73) Patentinhaber : **HOECHST AKTIENGESELLSCHAFT**
**Postfach 80 03 20**
**W-6230 Frankfurt am Main 80 (DE)**

(72) Erfinder : **Breipohl, Gerhard, Dr.**
**Geisenheimer Strasse 95**
**W-6000 Frankfurt am Main 71 (DE)**
Erfinder : **Knolle, Jochen, Dr.**
**Höchster Strasse 21**
**W-6239 Kriftel (DE)**
Erfinder : **Stüber, Werner, Dr.**
**Cölber Weg 12**
**W-3551 Lahntal (DE)**

EP 0 287 882 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Spacer und ihre Herstellungsverfahren sowie die Synthese von Peptidamiden mittels Festphasenmethode unter Verwendung dieser säurelabilen Ankergruppen.

Zur Herstellung von Peptidamiden mittels Festphasensynthese verwendet man im allgemeinen Benzhydrylamin- oder Methylbenzhydrylaminharze, wie sie z.B. bei J.P. Tam et al., Tetrahedron Lett. 22, 285 (1981) beschrieben sind. Eine weitere Methode besteht in der Ammonolyse von trägergebundenen Peptidbenzylestern (C. Ressler et al., J. Am. Chem. Soc. 76, 3107 (1951)). Beide Methoden sind charakterisiert durch die zur Abspaltung des "Spacers" notwendige starke Säure (flüssiger Fluorwasserstoff oder Trifluormethansulfonsäure), Nebenreaktionen oder unvollständige Abspaltung.

Der Erfindung liegt daher die Aufgabe zugrunde, neue Spacer zu finden, die eine mildere und bessere Abspaltung von Peptidamiden vom Trägerharz ermöglichen.

Diese Aufgabe wird erfindungsgemäß gelöst durch die Verbindungen der allgemeinen Formel

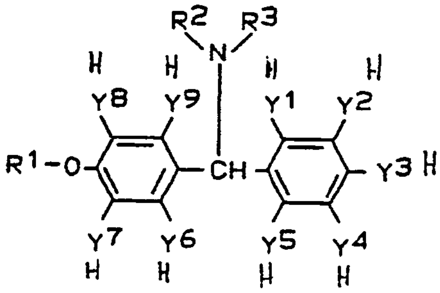

(I)

worin

$R^1$ $(C_1$-$C_8)$-Alkyl oder gegebenenfalls substituiertes $(C_6$-$C_{14})$-Aryl,

$R^2$ Wasserstoff oder ein Aminosäurerest, der mit einer schwach sauer oder basisch abspaltbaren Aminoschutzgruppe geschützt ist,

$R^3$ Wasserstoff oder $(C_1$-$C_4)$-Alkyl,

$Y^1$, $Y^2$, $Y^3$, $Y^4$, $Y^5$, $Y^6$, $Y^7$, $Y^8$, $Y^9$ Wasserstoff, $(C_1$-$C_4)$-Alkyl, $(C_1$-$C_4)$-Alkoxy oder $-O-(CH_2)_n-COOH$, wobei die Reste gleich oder verschieden sein können, ein Rest aber $-O-(CH_2)_n-COOH$ ist, und

n eine ganze Zahl von 1 bis 6

bedeuten.

Bevorzugt sind Verbindungen der allgemeinen Formel I worin $R^1$ Methyl und n eine ganze Zahl von 1, 2 oder 3 sind.

Ebenfalls bevorzugt sind Verbindungen dieser allgemeinen Formel I worin $R^2$ ein Aminosäurerest, der mit einer Urethanschutzgruppe – insbesondere Fmoc – geschützt ist, und $R^3$ Wasserstoff bedeuten.

Ferner stehen die Reste $Y^1$-$Y^9$ insbesondere für Methyl oder Methoxy, wobei jedoch ein Rest $-O-(CH_2)_n-COOH$ und mindestens 4 dieser Reste Wasserstoff sind.

Vorzugsweise stehen $Y^1$, $Y^3$, $Y^5$, $Y^7$ oder $Y^8$ für den Rest $-O-(CH_2)_n-COOH$.

Alkyl und Alkoxy können geradkettig oder verzweigt sein. $(C_6$-$C_{14})$-Aryl ist beispielsweise Phenyl, Naphthyl Biphenylyl oder Fluorenyl ; bevorzugt ist Phenyl.

$R^2$ steht für den Rest einer Aminosäure vorzugsweise einer α-Aminosäure, die, falls chiral, in der D– oder L-Form vorliegen kann. Bevorzugt sind Reste natürlich vorkommender Aminosäuren, deren Enantiomeren, Homologen, Derivate und einfache Metaboliten (vgl. z.B. Wünsch et al., Houben-Weyl 15/1 und 2, Stuttgart, Thieme 1974). So kommen beispielsweise in Frage :

Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, Cyta, Daad, Dab, Dadd, Dap, Dapm, Dasu, Djen, Dpa, Dtc, Fel, Gln, Glu, Gly, Guv, hCys, His, hSer, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val sowie die Reste der entsprechenden enantiomeren D-Aminosäuren.

Funktionelle Gruppen in den Seitenketten der genannten Aminosäurereste können geschützt vorliegen. Geeignete Schutzgruppen sind bei Hubbuch, Kontakte (Merck) 1979. Nr. 3, Seiten 14-23 und bei Bullesbach, Kontakte (Merck) 1980, Nr. 1, Seiten 23-35 beschrieben.

Basenlabile oder gegen schwache Säuren labile Schutzgruppen sind insbesondere Urethanschutzgruppen, wie Fmoc, Ddz, Bpoc, Msc, Peoc, Pse und Tse, vorzugsweise Fmoc (siehe z.B. Hubbuch, Kontakte

(Merck) 1979, Nr. 3, Seiten 14-23).

Die Erfindung betrifft auch ein Verfahren zur Herstellung der Verbindungen der Formel I, das dadurch gekennzeichnet ist, daß man

a) eine Verbindung der Formel II

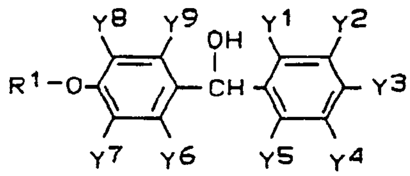

(II)

worin
$R^1$ $(C_1-C_8)$-Alkyl oder gegebenenfalls substituiertes $(C_6-C_{14})$-Aryl,
$Y^1$, $Y^2$, $Y^3$, $Y^4$, $Y^5$, $Y^6$, $Y^7$, $Y^8$, $Y^9$ Wasserstoff, $(C_1-C_4)$-Alkyl, $(C_1-C_4)$-Alkoxy oder $-O-(CH_2)_n-COOH$, wobei die Reste gleich oder verschieden sein können, ein Rest aber $-O-(CH_2)_n-COOH$ ist, und
n eine ganze Zahl von 1 bis 6
bedeuten,
mit einer Verbindung der Formel III

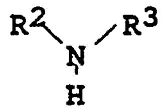

(III)

in welcher
$R^2$ Wasserstoff oder ein Aminosäurerest, der mit einer schwach sauer oder basisch abspaltbaren Aminoschutzgruppe geschützt ist, und
$R^3$ Wasserstoff oder $(C_1-C_4)$-Alkyl
bedeuten, umsetzt oder
b) eine Verbindung der Formel IV

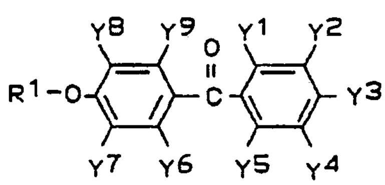

(IV)

mit Hydroxylamin zu einer Verbindung der Formel V

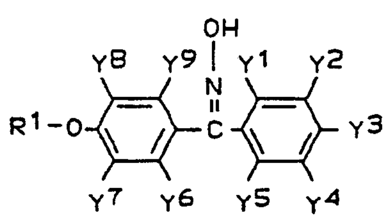

(V)

worin $R^1$, $Y^1$, $Y^2$, $Y^3$, $Y^4$, $Y^5$, $Y^6$, $Y^7$, $Y^8$ und $Y^9$ wie oben definiert sind, umsetzt
anschließend das Oxim zum Amin reduziert, vorzugsweise mit Zink in Eisessig (S. Gaehde, G. Matsueda,

Int. J. Peptide Protein Res. 18, 451 (1981)) und gegebenenfalls in seine Derivate überführt.

Die Umsetzung einer Verbindung der Formel III mit einer Verbindung der Formel II führt man vorzugsweise in einem polaren, protischen Lösungsmittel, wie z.B. Essigsäure bei einer Temperatur zwischen 0°C und dem Siedepunkt des Reaktionsgemisches durch.

Die Verbindungen der Formel II sind neu.

Verbindungen der Formel II erhält man beispielsweise durch Reduktion von Benzophenon-Derivaten der Formel IV,

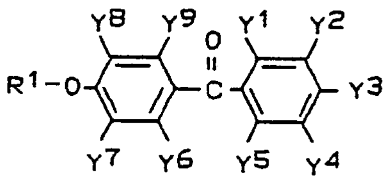

(IV)

worin $R^1$, $Y^1$, $Y^2$, $Y^3$, $Y^4$, $Y^5$, $Y^6$, $Y^7$, $Y^8$ und $Y^9$ wie oben definiert sind, mit geeigneten, d.h. für die Ketogruppe selektiven Reduktionsmitteln wie z.B. Natriumborhydrid.

Benzophenon-Derivate der Formel IV werden

a) durch Umsetzung von Benzophenonen der Formel IV worin $R^1$ wie oben definiert ist und $Y^1$, $Y^2$, $Y^3$, $Y^4$, $Y^5$, $Y^6$, $Y^7$, $Y^8$ und $Y^9$ Wasserstoff, ($C_1$-$C_4$)-Alkyl, ($C_1$-$C_4$)-Alkoxy und einer der Reste $Y^1$-$Y^9$ Hydroxyl bedeuten, mit ω-Halogenfettsäuren der Formel VI

$$Hal\text{-}(CH_2)_n\text{-}COOH \quad (VI)$$

worin Hal Halogen bedeutet und n wie oben definiert ist, oder deren Ester, wobei im Falle der Ester anschließend eine alkalische Verseifung der Estergruppierung z.B. mit Natronlauge erfolgt (M. Prashad et al., Indian J. Chem. 17B, 496-498 (1979)),

b) z.B. durch Umsetzung von Benzoesäurechloriden der Formel VII

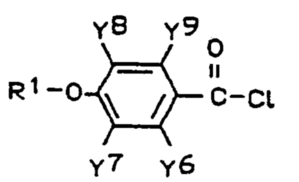

(VII)

mit ω-Phenoxyalkansäuren der Formel VIII

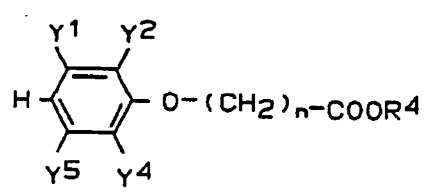

(VIII)

worin $R^1$, $Y^1$, $Y^2$, $Y^4$, $Y^5$, $Y^6$, $Y^7$, $Y^8$, $Y^9$ und n wie unter a) definiert sind und $R^4$ ($C_1$-$C_8$)-Alkyl, bevorzugt Methyl oder Ethyl bedeutet,

unter Verwendung eines Lewis-Katalysators wie z.B. Aluminiumtrichlorid oder Titantetrachlorid erhalten (Organikum, 13. Auflage, S. 354 (1974)), oder

c) durch Umsetzung der entsprechenden Benzoesäurechloride der Formel VII mit entsprechend substituierten Phenolen der Formel IX zu den entsprechenden Phenylestern der Formel X,

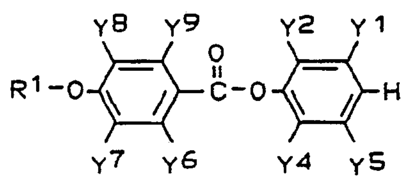

(X)

anschließende Fries-Verschiebung mit Lewis-Säuren wie z.B. Titantetrachlorid (R. Martin et al., Monatsh. Chemie 110, 1057-1066 (1979)) und weitere Umsetzung mit ω-Halogenfettsäuren der Formel VI, worin Hal und n wie oben definiert sind, erhalten.

Verbindungen der Formel VIII stellt man durch Umsetzung der entsprechenden Phenole der Formel IX

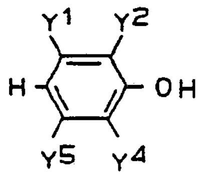

(IX)

mit den entsprechenden ω-Halogenalkansäureestern, z.B. mit Natriumhydrid in Dimethylformamid (DMF) oder Kaliumcarbonat in Aceton her.

Analog können auch die der allgemeinen Formel I entsprechenden Verbindungen der Formeln VII, VIII, IX, und X hergestellt werden.

Die Erfindung betrifft weiterhin die Verwendung einer Verbindung der Formel I, in welcher $R^2$ nicht Wasserstoff bedeutet, bei der Festphasensynthese von Verbindungen der Formel XI

$$P\text{-}R^2\text{-}NH\text{-}R^3 \quad (XI)$$

in welcher P für einen Peptidrest aus $q \leq p + 1$ α-Aminosäuren steht, $R^2$ ein Aminosäurerest, der mit einer schwach sauer oder basisch abspaltbaren Aminoschutzgruppe geschützt ist, bedeutet und $R^3$ wie oben definiert ist sowie ein Verfahren zur Herstellung eines Peptids der Formel XI, in welcher P, $R^2$ und $R^3$ wie oben definiert sind, durch Festphasensynthese, das dadurch gekennzeichnet ist, daß man eine Verbindung der Formel I mit in der Peptidchemie üblichen Kupplungsreagenzien über die $-O-(CH_2)_n-COOH$ Gruppierung an ein Harz kuppelt, die Schutzgruppe der Aminosäure $R^2$ abspaltet, stufenweise q-p durch basenlabile oder gegen schwache Säuren labile Aminoschutzgruppen temporär geschützte α-Aminosäuren gegebenenfalls in Form ihrer aktivierten Derivate ankuppelt und nach beendetem Aufbau das Peptid der Formel XI, durch Behandeln mit einer mittelstarken Säure von Harz freisetzt, wobei gleichzeitig oder durch geeignete Maßnahmen daran anschließend temporär eingeführte Seitenkettenschutzgruppen wieder abgespalten werden.

Falls zur Verhinderung von Nebenreaktionen oder für die Synthese spezieller Peptide erforderlich, sind die funktionellen Gruppen in der Seitenkette von Aminosäuren durch geeignete Schutzgruppen (siehe z.B. T.W. Greene, "Protective Groups in Organic Synthesis", New York, John Wiley & Sons, 1981) Zusätzlich geschützt, wobei in erster Linie Arg(Tos), Arg(Mts), Arg(Mtr), Asp(OBzl), Asp(OBut), Cys(4-MeBzl), Cys(Acm), Cys(SBut), Glu(OBzl), Glu(OBut), His(Tos), His(Fmoc), His(Dnp), His(Trt), Lys(Cl-2), Lys(Boc), Met(O), Ser(Bzl), Ser(But), Thr(Bzl), Thr(But) eingesetzt werden.

Die als Trägermaterial verwendeten Harze sind kommerziell erhältlich oder selbst hergestellt, wie z.B. Alkoxybenzylalkoholharze, Aminomethylharze oder Benzhydrylaminoharze. Bevorzugt sind Benzhydrylamino-(BHA) und Methylbenzhydrylamino-Harze (MBHA) Die Beladung bestimmt man durch Aminosäureanalyse und/oder Elementaranalyse.

Als Kupplungsreagenz für die Verbindung der Formel I und die weiteren Aminosäurederivate können alle möglichen in der Peptidsynthese verwendeten Aktivierungs-Reagenzien, siehe z.B. Houben-Weyl, Methoden der organischen Chemie, Band 15/2, verwendet werden, insbesondere aber Carbodiimide wie z.B. N,N'-Dicyclohexylcarbodiimid, N,N'-Diisopropylcarbodiimid oder N-Ethyl-N'-(3-dimethylaminopropyl)carbodiimid. Die Kupplung kann dabei direkt durch Addition von Aminosäurederivat mit dem Aktivierungsreagenz und gegebenenfalls einem die Racemisierung unterdrückenden Zusatz wie z.B. 4-Dimethylaminopyridin, 1-Hydroxybenzotriazol (HOBt) (W. König, R. Geiger, Chem. Ber. 103, 708 (1970)) oder

3-Hydroxy-4-oxo-3,4-dihydrobenzotriazin (HOObt) (W. König, R. Geiger, Chem. Ber. 103, 2054 (1970) zum Harz durchgeführt werden oder aber die Voraktivierung des Aminosäurederivats als symmetrisches Anhydrid oder HOBt- bzw. HOObt-Ester kann separat erfolgen und die Lösung der aktivierten Spezies in einem geeigneten Lösungsmittel zum kupplungsfähigen Peptidharz gegeben werden.

Die Kupplung bzw. Aktivierung der Verbindung der Formel I und der Aminosäurederivate mit einem der obengenannten Aktivierungsreagenzien kann in Dimethylformamid oder Methylenchlorid oder einer Mischung aus beiden durchgeführt werden. Das aktivierte Aminosäurederivat wird üblicherweise in einem 1,5- bis 4-fachen Überschuß eingesetzt. In Fällen, in denen eine unvollständige Kupplung eintritt, wird die Kupplungsreaktion wiederholt, ohne vorher die für die Kupplung der nächstfolgenden Aminosäure nötige Entblockung der α-Aminogruppe des Peptidharzes durchzuführen.

Der erfolgreiche Ablauf der Kupplungsreaktion kann mittels der Ninhydrin-Reaktion, wie z.B. von E. Kaiser et al. Anal. Biochem. 34 595 (1970) beschrieben, überprüft werden. Die Synthese kann auch automatisiert z.B. mit einem Peptid-Synthesizer Modell 430A der Fa. Applied Biosystems durchgeführt werden, wobei entweder die vom Gerätehersteller vorgesehenen Syntheseprogramme oder aber auch vom Benutzer selbst erstellte verwendet werden können. Letztere werden insbesondere bei der Verwendung von mit der Fmoc-Gruppe geschützten Aminosäurederivaten eingesetzt.

Die Abspaltung der Peptidamide vom Harz erfolgt durch Behandlung mit in der Peptidsynthese überlicherweise verwendeten mittelstarken Säuren (z.B. Trifluoressigsäure), wobei als Kationenfänger Substanzen wie Phenol, Kresol, Thiokresol, Anisol, Thioanisol, Ethandithiol, Dimethylsulfid, Ethylmethylsulfid oder ähnliche in der Festphasensynthese übliche Kationenfänger einzeln oder eine Mischung von zwei oder mehr dieser Hilfsmittel zugesetzt werden. Die Trifluoressigsäure kann dabei auch durch geeignete Lösungsmittel, wie z.B. Methylenchlorid, verdünnt angewendet werden.

Bei der Abspaltung des Spacers vom Harz erfolgt gleichzeitig die Abspaltung der Seitenkettenschutzgruppen.

Die Reinigung der so erhaltenen Rohpeptide erfolgt mittels Chromatographie an ®Sephadex, Ionenaustauscherharzen oder HPLC.

Die nachstehenden Beispiele dienen zur Erläuterung der vorstehenden Erfindung, ohne daß sie darauf beschränkt wäre.

**Beispiel 1**

4-(4′-Methoxybenzoyl)-phenoxyessigsäuremethylester

64 g Aluminiumchlorid (wasserfrei) werden in 160 ml 1.2-Dichlorethan gelöst und mit 71,6 g 4-Methoxybenzoylchlorid versetzt. Man tropft 57,6 ml Phenoxyessigsäuremethylester langsam unter Rühren hinzu und erhitzt das Reaktionsgemisch 4 Stunden auf 50°C. Der Ansatz wird auf Eiswasser getropft, wobei sich ein Öl abscheidet. Die Wasserphase wird abgetrennt, der Rückstand dreimal mit Wasser ausgerührt und mit Methanol kristallisiert. Der Niederschlag wurd abfiltriert und aus Ethylacetat umkristallisiert.

| | |
|---|---|
| Ausbeute : | 54,9 g (53% d. Th.) |
| Fp. : | 146°C (148°C, Ethylacetat) |

**Beispiel 2**

4-(4′-Methoxybenzoyl)-phenoxyessigsäure

9,0 g des Methylesters (Beispiel 1) werden in 120 ml 1.2-Dimethoxyethan/Wasser (4 : 1, v : v) gelöst und mit 15 ml 2N NaOH versetzt. Man rührt den Ansatz drei Stunden und stellt mit 3N HCl auf pH 3. Das organische Lösemittel wird im Vakuum abgedampft, das ausgefallene Produkt abfiltriert, mit Wasser gewaschen und im Hochvakuum getrocknet.

| | |
|---|---|
| Ausbeute : | 8,4 g (98% d. Th.) |
| Fp. : | 181-182°C |

**Beispiel 3**

(4- Carboxymethoxyphenyl)-4'-methoxyphenylcarbinol

11,2 g 4-(4'-Methoxybenzoyl)-phenoxyessigsäure werden in 600 ml 80%igem Methanol gelöst (Rückfluß) und mit 4,4 ml N-Methylmorpholin versetzt. Es werden portionsweise im Laufe von zwei Stunden 6 g Natriumborhydrid zugesetzt und die Reaktion 3 Stunden unter Rückflußbedingungen weitergeführt. Man kühlt den Ansatz auf Raumtemperatur ab und säuert mit 3N HCl auf pH 2,5 an. Das Methanol wird abdestilliert die wäßrige Phase mit Ethylacetat extrahiert, mit Kochsalzlösung gewaschen und über Natriumsulfat getrocknet. Nach Abdestillation des Ethylacetats bleibt ein weißes, amorphes Pulver. Das Produkt wird direkt in die nächste Reaktion eingesetzt.

Ausbeute : 9,3 g (83% d. Th.)

**Beispiel 4**

2-Methylphenoxyessigsäuremethylester

108 g 2-Methylphenol werden in 500 ml trockenem Aceton gelöst und 165,8 g gepulvertes Kaliumcarbonat zugesetzt. Zur gerührten Suspension gibt man 113 ml Bromessigsäuremethylester und läßt bei Raumtemperatur unter Feuchtigkeitsausschluß weiterrühren. Nach beendeter Reaktion wird vom Salz abgesaugt, mit Aceton nachgewaschen und das Filtrat eingeengt. Man nimmt in Ethylacetat auf, wäscht mit Wasser, trocknet die organische Phase über Magnesiumsulfat und engt ein.

Ausbeute : 180 g einer öligen Flüssigkeit, die direkt weiter umgesetzt wird.

**Beispiel 5**

4-(4'-Methoxybenzoyl)-2-methylphenoxyessigsäuremethylester

146,6 g wasserfreies Aluminiumtrichlorid werden in 500 ml 1.2-Dichlorethan gelöst. Bei 0°C tropft man nacheinander 187 g 4-Methoxybenzoylchlorid und 180 g 2-Methylphenoxyessigsäuremethylester hinzu. Zur Vervollständigung der Reaktion wird auf 50°C erhitzt. Der Ansatz wird auf Eis gegossen und mit 2N HCl auf pH 2 gestellt. Das ausgefallene Produkt wird abgesaugt und mit Wasser und etwas Ether gewaschen. Der Niederschlag wird in Ethylacetat unter Zugabe von etwas Aktivkohle heiß gelöst, filtriert und bei −10°C kristallisiert. Das Produkt wird abgesaugt, mit Ether gewaschen und im Hochvakuum getrocknet.

Ausbeute : 172,8 g (55% d. Th.)
Fp. : 92-95°C

**Beispiel 6**

4-(4'-Methoxybenzoyl)-2-methylphenoxyessigsäuremethylester

50 g 4-Hydroxy-3-methyl-4'-methoxybenzophenon (R. Martin et al. Monatsh. Chemie 110, 1057-1066 (1979)) werden in 200 ml trockenem DMF gelöst und unter $N_2$ vorsichtig mit 9 g einer 55%igen Dispersion von Natriumhydrid in Mineralöl versetzt. Dann tropft man unter Rühren 19,5 ml Bromessigsäuremethylester zu und läßt über Nacht bei Raumtemperatur stehen. Man saugt von ausgefallenem Salz ab und engt das Filtrat im Vakuum ein. Der Rückstand wird in Ethylacetat aufgenommen und mit Natriumhydrogencarbonat-Lösung und Wasser gewaschen. Man trocknet die organische Phase über Magnesiumsulfat und zieht das Lösungsmittel ab. Der Rückstand wird mit Ether verrieben, abgesaugt und getrocknet.

Ausbeute : 38,9 g (60% d. Th.)
Fp. : 96-98°C

**Beispiel 7**

4-(4'-Methoxybenzoyl)-phenoxyessigsäuremethylester

29,1 g 4-Hydroxy-4'-methoxybenzophenon (R.Martin et al. Monatsh. Chemie 110, 1057-1066 (1979)) werden in 400 ml trockenem Aceton gelöst. Anschließend gibt man unter Rühren 19,3 g feingepulvertes $K_2CO_3$ und 16 ml Bromessigsäuremethylester zu und rührt bei Raumtemperatur. Nach 2 Tagen ist die Reaktion beendet. Man saugt von ausgefallenem Salz-Substanzgemisch ab und engt das Filtrat ein. Beide Rückstände werden in Wasser suspendiert und mit 2N HCl auf pH 3 gestellt. Man saugt ab, wäscht mit Wasser und trocknet im Exsiccator im Hochvakuum.

Ausbeute : 35,7 g (98% d. Th.)
Fp. : 143-145°C

**Beispiel 8**

4-(4'-Methoxybenzoyl)-2-methylphenoxyessigsäure

35,8 g 4-(4'-Methoxybenzoyl)-2-methylphenoxyessigsäuremethylester werden mit einer Mischung aus 240 ml Dioxan und 240 ml 0,5 N NaOH bei Raumtemperatur gerührt. Nach beendeter Reaktion wird das organische Lösemittel abgezogen, die wäßrige Phase mit 2N HCl auf pH 3 gestellt und mit Ethylacetat extrahiert. Man wäscht mit Wasser, trocknet über Magnesiumsulfat und engt ein. Es verbleiben blaßgelbe Kristalle.

Ausbeute : 30,2 g (83% d. Th.)
Fp. : 149-151°C

**Beispiel 9**

(4-Carboxymethoxy-3-methylphenyl)-4'-methoxyphenylcarbinol

22,5 g 4-(4'-Methoxybenzoyl)-2-methylphenoxyessigsäure werden in einer Mischung von 100 ml Dioxan und 200 ml Wasser unter Zugabe von 1N NaOH bis pH 9 gelöst. Zur gerührten Lösung werden portionsweise 2,8 g Natriumtorhydrid gegeben und über Nacht stehengelassen. Anschließend wird das Dioxan abgezogen, die Wasserphase mit 2N HCl auf pH 3 gestellt und mit Ethylacetat extrahiert. Die organische Phase wird über Magnesiumsulfat getrocknet und eingeengt. Es verbleibt ein farbloser Schaum der mit n-Hexan zu einem amorphen Pulver verrieben und dann abgesaugt wird. Das Produkt wird direkt in die nächste Reaktion eingesetzt.

Ausbeute : 19,2 g (84% d. Th.)

**Beispiel 10**

2.6-Dimethylphenoxyessigsäuremethylester

65 g 2.6-Dimethylphenol werden in 200 ml trockenem DMF gelöst und unter $N_2$ portionsweise mit 23,2 g einer 55%igen Suspension von Natriumhydrid in Mineralöl versetzt. Dann werden 50,4 ml Bromessigsäuremethylester unter Rühren zugetropft und die Mischung noch über Nacht stehengelassen. Man saugt von ausgefallenem Salz ab und engt das Filtrat ein. Der Rückstand wird in Ethylacetat aufgenommen und mit Wasser extrahiert. Die organische Phase wird über Magnesiumsulfat getrocknet und eingeengt. Es verbleibt eine ölige Flüssigkeit, die direkt in die folgende Reaktion eingesetzt wird.

Ausbeute : 95,8 g (92% d. Th.)

**Beispiel 11**

2.6-Dimethyl-4-(4'-methoxybenzoyl)-phenoxyessigsäuremethylester

Die Synthese erfolgt analog Beispiel 5 mit 19,4 g 2.6-Dimethylphenoxyessigsäuremethylester.

Ausbeute : 17,4 g (53% d. Th.)

**Beispiel 12**

(4-Carboxymethoxy -3.5-dimethylphenyl)-4′-methoxyphenylcarbinol

16,4 g 2.6-Dimethyl-4-(4′-methoxybenzoyl)-phenoxyessigsäuremethylester werden in einer Mischung von 100 ml 0,5 N NaOH und 100 ml Dioxan bei Raumtemperatur gerührt. Nach beendeter Verseifung des Methylesters werden 1,89 g Natriumborhydrid zugesetzt und über Nacht reagieren gelassen. Auschließend wird ein wenig ungelöstem abgesaugt, das Filtrat eingeengt und die verbleibende wäßrige Lösung mit 1N HCl angesäuert. Man extrahiert mit Ethylacetat, wäscht die organische Phase mit Wasser, trocknet über Magnesiumsulfat und engt ein. Es verbleibt ein amorphes Pulver, das für die nachfolgende Umsetzung direkt eingesetzt wird.

Ausbeute : 11,3 g (71% d. Th.)

Allgemeine Vorschrift zur Herstellung der Nα-Fmoc-Aminosäure-(4-carboxymethoxy-phenyl-4′-methoxyphenyl)methylamide sowie der Nα-Fmoc-Aminosäure-(4-carboxymethoxy-3-methylphenyl-4′-methoxyphenyl)-methylamide und der Nα-Fmoc-Aminosäure-(4-carboxymethoxy-3.5-dimethylphenyl-4′-methoxyphenyl)-methylamide.

10 mMol Nα-Fmoc-Aminosäureamid und 10 mMol des entsprechenden Carbinols werden in der notwendigen Menge wasserfreien Eisessigs gelöst und mit 5-10 Tropfen konzentrierter Schwefelsäure versetzt. Man gibt noch 2 g Molekularsieb hinzu und läßt über Nacht stehen. Anschließend saugt man vom Molekularsieb ab und verdünnt das Filtrat mit viel Wasser wobei das Produkt zum Teil ausfällt. Man extrahiert die wäßrige Phase mit Ethylacetat und schüttelt dann die organische Phase mit Wasser aus. Die nach Trocknen über Magnesiumsulfat und Einengen verbleibende Substanz wird umkristallisiert.

Nach obiger allgemeinen Vorschrift wurden folgende Verbindungen hergestellt :

**Beispiel 13**

Nα-Fmoc-Glycin-(4-carboxymethoxyphenyl-4′-methoxyphenyl)methylamid

Ausbeute : 65%
Fp. : 136-138°C

**Beispiel 14**

Nα-Fmoc-Phenylalanin-(4-carboxymethoxyphenyl-4′methoxyphenyl)methylamid

Ausbeute : 65%
Fp. : 159-162°C

**Beispiel 15**

Nα-Fmoc-Glycin-(4-carboxymethoxy-3-methylphenyl-4′-methoxyphenyl)methylamid

Ausbeute : 60%
Fp. : 135-140°C

**Beispiel 16**

Nα-Fmoc-Valin-(4-carboxymethoxy-3-methylphenyl-4′-methoxyphenyl)methylamid

Ausbeute : 81%
Fp. : 172-175°C

**Beispiel 17**

Nα-Fmoc-Glycin-(4-carboxymethoxy-3.5-dimethylphenyl-4'-methoxyphenyl)methylamid

Ausbeute : 70%
Fp. : 122-126°C

**Beispiel 18**

Synthese von Oxytocin

H-Cys-Tyr-Ile-Gln-Asn-Cys-Pro-Leu-Gly-$NH_2$

unter Verwendung des in Beispiel 13 beschriebenen Ankers. Die Synthese erfolgte in einem Peptidsynthesizer der Fa. Labotec.

Von 1,5 g Boc-Val-Harz (Beladung 0,76 mMol/g) wird zunächst mit Trifluoressigsäure in Methylenchlorid die Schutzgruppe entfernt. Nach dem Waschen mit Dichlormethan und Ethyldiisopropylamin und wiederum Dichlormethan wird das Harz getrocknet. Anschließend gibt man 2,1 mMol des in Beispiel 13 hergestellten Ankers zusammen mit 3,15 mMol HOBt gelöst in 20 ml trockenem DMF auf das Harz und fügt 2,3 mMol Diisopropylcarbodiimid hinzu. Unter langsamen Durchmischen läßt man bei Raumtemperatur über Nacht reagieren. Die Vollständigkeit der Reaktion wird mit der Ninhydrin-Reaktion (Kaiser-Test) überprüft. Dann wird das Harz abgesaugt, mit DMF gewaschen und anschließend das Peptid am Harz aufgebaut, wobei man folgende Schritte cyclisch durchläuft :

- Abspaltung der Fmoc-Schutzgruppe mit 20% Piperidin in DMF
- Waschen des Harzes mit DMF
- Aufkuppeln der Fmoc-Aminosäure unter in situ Aktivierung als HOBt-Ester unter Verwendung von Diisopropylcarbodiimid als Aktivierungsreagenz (2,1 mMol Aminosäure, 3,15 mMol HOBt, 2,3 mMol Diisopropylcarbodiimid)
- Waschen des Harzes mit DMF.

Falls die Kupplung unvollständig ist (Kaiser-Test), wird der Kupplungsschritt wiederholt.

Als Seitenkettenschutzgruppen verwendet man tert.-Butyl für Tyrosin und tert.-Butylthio für Cystein.

Nachbeendeter-Synthese wird zunächst noch die Fmoc-Schutzgruppe abgespalten und dann das Harz nacheinander mit DMF, Dichlormethan, Isopropanol, Dichlormethan und tert.Butyl-methylether gewaschen und im Hochvakuum getrocknet.

Man erhält 2,4 g Peptidharz.

Die Abspaltung erfolgt bei Raumtemperatur mit einer Mischung aus Trifluoressigsäure/Thioanisol/Ethandithiol (80/15/5). Nach 2 Stunden wird in tert.-Butyl-methylether abgesaugt, das ausgefallene Rohpeptid abzentrifugiert und dreimal mit tert.Butyl-methylether gewaschen. Die Abspaltung der tert.Butylthio-Schutzgruppe erfolgt durch Tributylphosphin in Trifluorethanol/Wasser bei pH 7,3. Das S-H Peptid wird mit Jod in 60%iger Essigsäure cyclisiert und durch Chromatographie an ®Sephadex LH 20 in Methanol gereinigt.

Ausbeute an Oxytocin 33%, identisch mit einer authentischen Vergleichsprobe.

## Ansprüche

### Patentansprüche für die benannten Vertrasstaaten : AT, BE, CH, DE, FR, GB, GR, IT, LI, LU, NL, SE

1. Verbindung der Formel I,

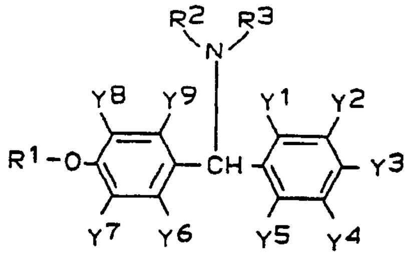

(I)

in welcher
$R^1$ $(C_1-C_8)$-Alkyl oder gegebenenfalls substituiertes $(C_6-C_{14})$-Aryl,
$R^2$ Wasserstoff oder ein Aminosäurerest, der mit einer schwach sauer oder basisch abspaltbaren Aminoschutzgruppe geschützt ist,
$R^3$ Wasserstoff oder $(C_1-C_4)$-Alkyl,
$Y^1$, $Y^2$, $Y^3$, $Y^4$, $Y^5$, $Y^6$, $Y^7$, $Y^8$, $Y^9$ Wasserstoff, $(C_1-C_4)$-Alkyl, $(C_1-C_4)$-Alkoxy oder $-O-(CH_2)_n-COOH$, wobei die Reste gleich oder verschieden sein können, ein Rest aber $-O-(CH_2)_n-COOH$ ist, und
n eine ganze Zahl von 1 bis 6
bedeuten.

2. Verbindung der Formel I gemäß Anspruch 1, in welcher $R^1$ Methyl und n eine ganze Zahl von 1, 2 oder 3 sind.

3. Verbindung der Formel I gemäß einem oder mehreren der Ansprüche 1 bis 2, in welcher $R^2$ ein Aminosäurerest, der mit einer Urethanschutzgruppe geschützt ist, und $R^3$ Wasserstoff bedeuten.

4. Verbindung der Formel I gemäß einem oder mehreren der Ansprüche 1 bis 3, in welcher die Reste $Y^1$, $Y^2$, $Y^3$, $Y^4$, $Y^5$, $Y^6$, $Y^7$, $Y^8$ und $Y^9$ für Methyl oder Methoxy stehen, wobei jedoch einer dieser Reste $-O-(CH_2)_n-COOH$ und mindestens 4 dieser Reste Wasserstoff bedeuten.

5. Verbindung der Formel I gemäß einem oder mehreren der Ansprüche 1 bis 4, in welcher die Reste $Y^1$, $Y^3$, $Y^5$, $Y^7$ oder $Y^8$ für den Rest $-O-(CH_2)_n-COOH$ stehen.

6. Verfahren zur Herstellung einer Verbindung der Formel I gemäß einem oder mehreren der Ansprüche 1 bis 5, dadurch gekennzeichnet, daß man

a) eine Verbindung der Formel II,

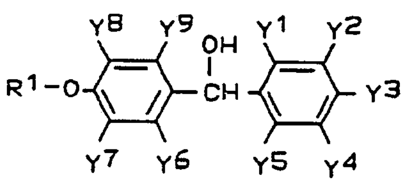

(II)

in welcher
$R^1$ $(C_1-C_8)$-Alkyl oder gegebenenfalls substituiertes $(C_6-C_{14})$-Aryl,
$Y^1$, $Y^2$, $Y^3$, $Y^4$, $Y^5$, $Y^6$, $Y^7$, $Y^8$, $Y^9$ Wasserstoff, $(C_1-C_4)$-Alkyl, $(C_1-C_4)$-Alkoxy oder $-O-(CH_2)_n-COOH$, wobei die Reste gleich oder verschieden sein können, ein Rest aber $-O-(CH_2)_n-COOH$ ist, und
n eine ganze Zahl von 1 bis 6
bedeuten,
mit einer Verbindung der Formel III,

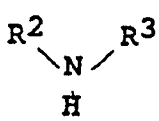

(III)

in welcher
$R^2$ Wasserstoff oder ein Aminosäurerest, der mit einer schwach sauer oder basisch abspaltbaren Aminoschutzgruppe geschützt ist, und
$R^3$ Wasserstoff oder $(C_1-C_4)$-Alkyl bedeuten, umsetzt oder

b) eine Verbindung der Formel IV,

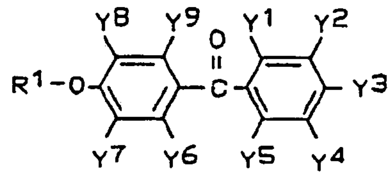

(IV)

mit Hydroxylamin zu einer Verbindung der Formel V,

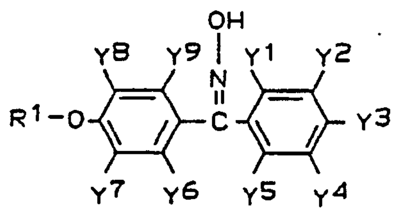

(V)

worin $R^1$, $Y^1$, $Y^2$, $Y^3$, $Y^4$, $Y^5$, $Y^6$, $Y^7$, $Y^8$ und $Y^9$ wie unter a) definiert sind, umsetzt, anschließend das Oxim zum Amin reduziert und gegebenenfalls in seine Derivate überführt.

7. Verwendung einer Verbindung der Formel I gemäß einem oder mehreren der Ansprüche 1 bis 5, in welcher $R^2$ nicht Wasserstoff bedeutet, bei der Festphasensynthese von Verbindungen der Formel XI,

$$P\text{-}R^2\text{-}NH\text{-}R^3 \quad (XI)$$

in welcher P für einem Peptidrest aus $q \leq p + 1$ α-Aminosäuren steht, $R^2$ ein Aminosäurerest bedeutet und $R^3$ wie in Anspruch 1 definiert ist.

8. Verfahren zur Herstellung eines Peptids der Formel XI, in welcher P, $R^2$ und $R^3$ wie in Anspruch 7 definiert sind, durch Festphasensynthese, dadurch gekennzeichnet, daß man eine Verbindung der Formel I gemäß einem oder mehreren der Ansprüch 1 bis 5, in welcher $R^2$ nicht Wasserstoff bedeutet, an ein Harz kuppelt, die Schutzgruppe der Aminosäure $R^2$ abspaltet, stufenweise q-p durch basenlabile oder gegen schwache Säuren labile Aminoschutzgruppen temporär geschützte α-Aminosäuren gegebenenfalls in Form ihrer aktivierten Derivate ankuppelt und nach beendetem Aufbau das Peptid der Formel XI, durch Behandeln mit einer mittelstarken Säure von Harz freigesetzt, wobei gleichzeitig oder durch geeignete Maßnahmen daran anschließend temporär eingeführte Seitenkettenschutzgruppen wieder abgespalten werden.

9. Verbindung der Formel II,

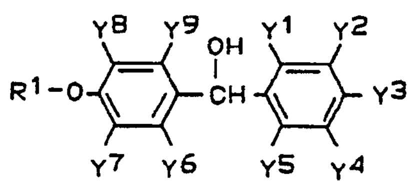

(II)

in welcher
$R^1$ ($C_1$-$C_8$)-Alkyl oder gegebenenfalls substituiertes ($C_6$-$C_{14}$)-Aryl,
$Y^1$, $Y^2$, $Y^3$, $Y^4$, $Y^5$, $Y^6$, $Y^7$, $Y^8$, $Y^9$ Wasserstoff, ($C_1$-$C_4$)-Alkyl, ($C_1$-$C_4$)-Alkoxy oder $-O-(CH_2)_n-COOH$, wobei die Reste gleich oder verschieden sein können, ein Rest aber $-O-(CH_2)_n-COOH$ ist, und
n eine ganze Zahl von 1 bis 6
bedeuten.

**Patentansprüche für den benannten Vertragsstaat : ES**

1. Verfahren zur Herstellung einer Verbindung der Formel I,

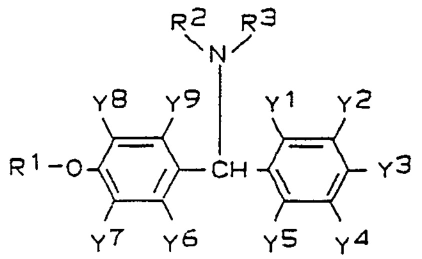

(I)

in welcher
$R^1$ ($C_1$-$C_8$)-Alkyl oder gegebenenfalls substituiertes ($C_6$-$C_{14}$)-Aryl, $R^2$ Wasserstoff oder ein Aminosäurerest, der mit einer schwach sauer oder basisch abspaltbaren Aminoschutzgruppe geschützt ist,
$R^3$ Wasserstoff oder ($C_1$-$C_4$) Alkyl,
$Y^1$, $Y^2$, $Y^3$, $Y^4$, $Y^5$, $Y^6$, $Y^7$, $Y^8$, $Y^9$ Wasserstoff, ($C_1$-$C_4$)-Alkyl, ($C_1$-$C_4$)-Alkoxy oder $-O-(CH_2)_n-COOH$, wobei die Reste gleich oder verschieden sein können, ein Rest aber $-O-(CH_2)_n-COOH$ ist, und
n eine ganze Zahl von 1 bis 6
bedeuten, dadurch gekennzeichnet, daß man

a) eine Verbindung der Formel II,

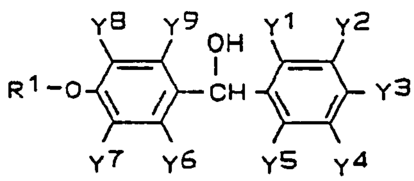

(II)

in welcher
$R^1$ ($C_1$-$C_8$)-Alkyl oder gegebenenfalls substituiertes ($C_6$-$C_{14}$)-Aryl,
$Y^1$, $Y^2$, $Y^3$, $Y^4$, $Y^5$, $Y^6$, $Y^7$, $Y^8$, $Y^9$ Wasserstoff, ($C_1$-$C_4$)-Alkyl, ($C_1$-$C_4$)-Alkoxy oder $-O-(CH_2)_n-COOH$, wobei die Reste gleich oder verschieden sein können, ein Rest aber $-O-(CH_2)_n-COOH$ ist, und
n eine ganze Zahl von 1 bis 6
bedeuten,
mit einer Verbindung der Formel III,

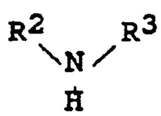

(III)

in welcher
$R^2$ Wasserstoff oder ein Aminosäurerest, der mit einer schwach sauer oder basisch abspaltbaren Aminoschutzgruppe geschützt ist, und
$R^3$ Wasserstoff oder ($C_1$-$C_4$)-Alkyl
bedeuten, umsetzt oder

b) eine Verbindung der Formel IV,

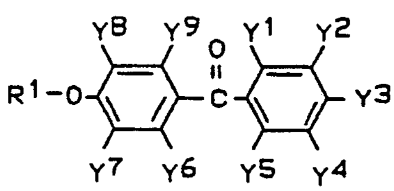

(IV)

mit Hydroxylamin zu einer Verbindung der Formel V,

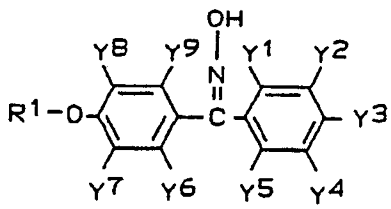

(V)

worin $R^1$, $Y^1$, $Y^2$, $Y^3$, $Y^4$, $Y^5$, $Y^6$, $Y^7$, $Y^8$ und $Y^9$ wie unter a) definiert sind, umsetzt, anschließend das Oxim zum Amin reduziert und gegebenenfalls in seine Derivate überführt.

2. Verfahren zur Herstellung einer Verbindung der Formel I gemäß Anspruch 1, in welcher $R^1$ Methyl und n eine ganze Zahl von 1, 2 oder 3 sind.

3. Verfahren zur Herstellung einer Verbindung der Formel I gemäß einem oder mehreren der Ansprüche 1 bis 2, in welcher $R^2$ ein Aminosäurerest, der mit einer Urethanschutzgruppe geschützt ist, und $R^3$ Wasserstoff bedeuten.

4. Verfahren zur Herstellung einer Verbindung der Formel I gemäß einem oder mehreren der Ansprüche 1 bis 3, in welcher die Reste $Y^1$, $Y^2$, $Y^3$, $Y^4$, $Y^5$, $Y^6$, $Y^7$, $Y^8$ und $Y^9$ für Methyl oder Methoxy stehen, wobei jedoch einer dieser Reste $-O-(CH_2)_n-COOH$ und mindestens 4 dieser Reste Wasserstoff bedeuten.

5. Verfahren zur Herstellung einer Verbindung der Formel I gemäß einem oder mehreren der Ansprüche 1 bis 4, in welcher die Reste $Y^1$, $Y^3$, $Y^5$, $Y^7$ oder $Y^8$ für den Rest $-O-(CH_2)_n-COOH$ stehen.

6. Verfahren zur Herstellung eines Peptids der Formel XI,

$$P\text{-}R^2\text{-}NH\text{-}R^3 \quad (XI)$$

in welcher P für einem Peptidrest aus $q \leq p + 1$ α-Aminosäuren steht, $R^2$ ein Aminosäurerest bedeutet und $R^3$ wie in Anspruch 1 definiert ist, durch Festphasensynthese, dadurch gekennzeichnet, daß man eine Verbindung der Formel I gemäß einem oder mehreren der Ansprüch 1 bis 5, in welcher $R^2$ nicht Wasserstoff bedeutet, an ein Harz kuppelt, die Schutzgruppe der Aminosäure $R^2$ abspaltet, stufenweise q-p durch basenlabile oder gegen schwache Säuren labile Aminoschutzgruppen temporär geschützte α-Aminosäuren gegebenenfalls in Form ihrer aktivierten Derivate ankuppelt und nach beendetem Aufbau das Peptid der Formel XI, durch Behandeln mit einer mittelstarken Säure von Harz freigesetzt, wobei gleichzeitig oder durch geeignete Maßnahmen daran anschließend temporär eingeführte Seitenkettenschutzgruppen wieder abgespalten werden.

7. Verfahren zur Herstellung einer Verbindung der Formel II,

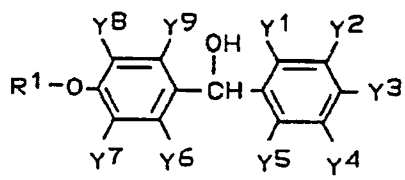

(II)

in welcher

$R^1$ $(C_1\text{-}C_8)$-Alkyl oder gegebenenfalls substituiertes $(C_6\text{-}C_{14})$-Aryl,

$Y^1$, $Y^2$, $Y^3$, $Y^4$, $Y^5$, $Y^6$, $Y^7$, $Y^8$, $Y^9$ Wasserstoff, $(C_1\text{-}C_4)$-Alkyl, $(C_1\text{-}C_4)$-Alkoxy oder $-O-(CH_2)_n-COOH$, wobei die Reste gleich oder verschieden sein können, ein Rest aber $-O-(CH_2)_n-COOH$ ist, und

n eine ganze Zahl von 1 bis 6

bedeuten, dadurch gekennzeichnet, daß man Benzophenon-Derivate der Formel IV,

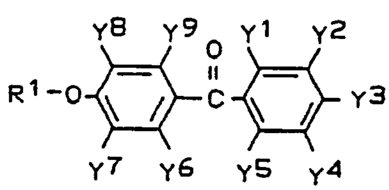

(IV)

worin $R^1$, $Y^1$, $Y^2$, $Y^3$, $Y^4$, $Y^5$, $Y^6$, $Y^7$, $Y^8$ und $Y^9$ wie oben definiert sind, reduziert.

## Claims

### C laims for the Contracting States AT, BE, DE, FR, GB, GR, IT, LI, LU, NL, SE

1. A compound of the formula I

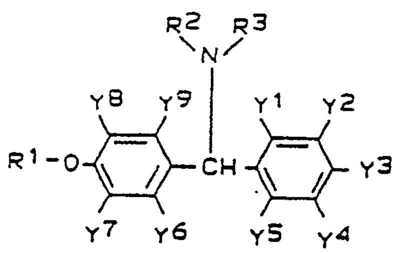

(I)

in which
$R^1$ denotes $(C_1-C_8)$-alkyl or optionally substituted $(C_6-C_{14})$-aryl,
$R^2$ denotes hydrogen or an amino acid residue which is protected by an amino protective group which can be cleaved off with weak acid or base,
$R^3$ denotes hydrogen or $(C_1-C_4)$ -alkyl,
$Y^1$, $Y^2$, $Y^3$, $Y^4$, $Y^5$, $Y^6$, $Y^7$, $Y^8$ and $Y^9$ denote hydrogen, $(C_1-C_4)$ -alkyl, $(C_1-C_4)$ -alkoxy or $-O(CH_2)_n$-COOH, it being possible for the radicals to be identical or different but one radical being $-O-(CH_2)_n$-COOH, and
n denotes an integer from 1 to 6.

2. A compound of the formula I as claimed in claim 1, in which $R^1$ is methyl, and n is an integer 1, 2 or 3.

3. A compound of the formula I as claimed in one or more of claims 1 and 2, in which $R^2$ denotes an amino acid residue which is protected with a urethane protective group, and $R^3$ denotes hydrogen.

4. A compound of the formula I as claimed in one or more of claims 1 to 3, in which the radicals $Y^1$, $Y^2$, $Y^3$, $Y^4$, $Y^5$, $Y^6$, $Y^7$, $Y^8$ and $Y^9$ represent methyl or methoxy, with, however, one of these radicals denoting $-O(CH_2)_n$-COOH and at least 4 of these radicals denoting hydrogen.

5. A compound of the formula I as claimed in one or more of claims 1 to 4, in which the radicals $Y^1$, $Y^3$, $Y^5$ , $Y^7$ or $Y^8$ represent the radical $-O-(CH_2)_n$-COOH.

6. A process for the preparation of a compound of the formula I as claimed in one or more of claims 1 to 5, which comprises

a) reaction of a compound of the formula II

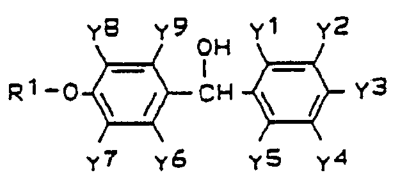

(II)

in which
$R^1$ denotes $(C_1-C_8)$-alkyl or optionally substituted $(C_6-C_{14})$-aryl,
$Y^1$ , $Y^2$, $Y^3$, $Y^4$, $Y^5$, $Y^6$, $Y^7$, $Y^8$ and $Y^9$ denote hydrogen, $(C_1-C_4)$-alkyl, $(C_1-C_4)$-alkoxy or $-O-(CH_2)_n$-COOH, it being possible for the radicals to be identical or different but one radical being $-O-(CH_2)_n$-COOH, and
n denotes an integer from 1 to 6,
with a compound of the formula III

(III)

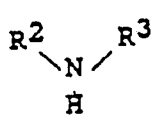

in which
$R^2$ denotes hydrogen or an amino acid residue which is protected by an amino protective group which can

be cleaved off by weak acid or base, and
$R^3$ denotes hydrogen or ($C_1$-$C_4$)-alkyl, or
b) reaction of a compound of the formula IV

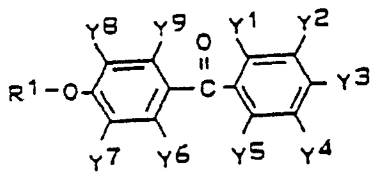

(IV)

with hydroxylamine to give a compound of the formula V

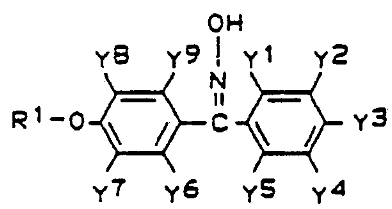

(V)

in which $R^1$, $Y^1$, $Y^2$, $Y^3$, $Y^4$, $Y^5$, $Y^6$, $Y^7$, $Y^8$ and $Y^9$ are as defined under a), then reduction of the oxime to the amine and, where appropriate, conversion into its derivatives.

7. The use of a compound of the formula I as claimed in one or more of claims 1 to 5, in which $R^2$ does not denote hydrogen, in the solid phase synthesis of compounds of the formula XI

$$P-R^2-NH-R^3 \qquad (XI)$$

in which P represents a peptide residue composed of $q \leq p + 1$ α-amino acids, $R^2$ denotes an amino acid residue, and $R^3$ is as defined in claim 1.

8. A process for the preparation of a peptide of the formula XI, in which P, $R^2$ and $R^3$ are as defined in claim 7, by solid phase synthesis, which comprises coupling a compound of the formula I as claimed in one or more of claims 1 to 5, in which $R^2$ does not denote hydrogen, to a resin, cleaving off the protective group on the amino acid $R^2$, stepwise coupling on of q-p a-amino acids which are, where appropriate, in the form of their activated derivatives and have been temporarily protected by amino protective groups which are base-labile or labile to weak acids, and, after the synthesis is complete, liberation of the peptide of the formula XI from the resin by treatment with a moderately strong acid, with temporarily introduced sidechain protective groups being cleaved off again at the same time or by suitable measures subsequent thereto.

9. A compound of the formula II,

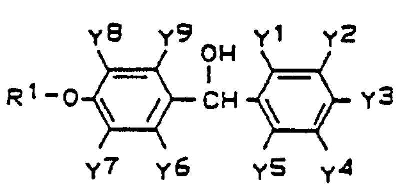

(II)

in which
$R^1$ denotes ($C_1$-$C_8$) -alkyl or optionally substituted ($C_6$-$C_{14}$)-aryl,
$Y^1$, $Y^2$, $Y^3$, $Y^4$, $Y^5$, $Y^6$, $Y^7$, $Y^8$ and $Y^9$ denote hydrogen, ($C_1$-$C_4$)-alkyl, ($C_1$-$C_4$)-alkoky or $-O-(CH_2)_n-COOH$, it being possible for the radicals to be identical or different but one radical being $-O-(CH_2)_n-COOH$, and
n denotes an integer from 1 to 6.

**Claims for the Contracting State ES**

1. A process for the preparation of a compound of the formula I

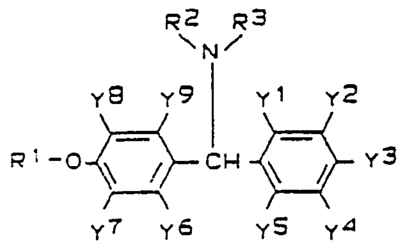

(I)

in which
$R^1$ denotes $(C_1-C_8)$-alkyl or optionally substituted $(C_6-C_{14})$-aryl,
$R^2$ denotes hydrogen or an amino acid residue which is protected by an amino protective group which can be cleaved off with weak acid or base,
$R^3$ denotes hydrogen or $(C_1-C_4)$-alkyl,
$Y^1$, $Y^2$, $Y^3$, $Y^4$, $Y^5$, $Y^6$, $Y^7$, $Y^8$ and $Y^9$ denote hydrogen, $(C_1-C_4)$-alkyl, $(C_1-C_4)$-alkoxy or $-O-(CH_2)_n-COOH$, it being possible for the radicals to be identical or different but one radical being $-O-(CH_2)_n-COOH$, and
n denotes an integer from 1 to 6,
which comprises

a) reaction of a compound of the formula II

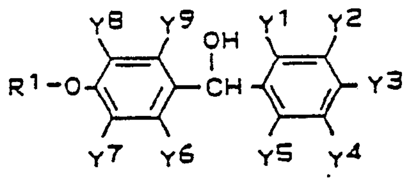

(II)

in which
$R^1$ denotes $(C_1-C_8)$-alkyl or optionally substituted $(C_6-C_{14})$-aryl,
$Y^1$ , $Y^2$, $Y^3$, $Y^4$, $Y^5$, $Y^6$, $Y^7$, $Y^8$ and $Y^9$ denote hydrogen, $(C_1-C_4)$-alkyl, $(C_1-C_4)$-alkoxy or $-O-(CH_2)_n-COOH$, it being possible for the radicals to be identical or different but one radical being $-O-(CH_2)_n-COOH$, and
n denotes an integer from 1 to 6,
with a compound of the formula III

(III)

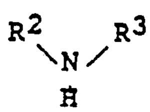

in which
$R^2$ denotes hydrogen or an amino acid residue which is protected by an amino protective group which can be cleaved off by weak acid or base, and
$R^3$ denotes hydrogen or $(C_1-C_4)$-alkyl, or

b) reaction of a compound of the formula IV

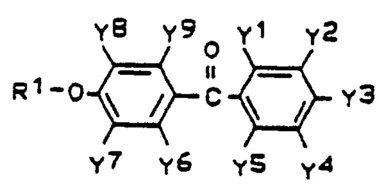

(IV)

with hydroxylamine to give a compound of the formula V

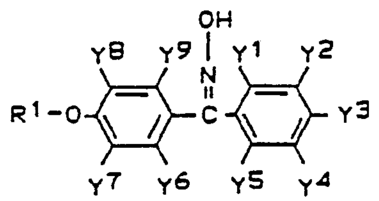

(V)

in which $R^1$, $Y^1$, $Y^2$, $Y^3$, $Y^4$, $Y^5$, $Y^6$, $Y^7$, $Y^8$ and $Y^9$ are as defined under a), then reduction of the oxime to the amine and, where appropriate, conversion into its derivatives.

2. The process for the preparation of a compound of the formula I as claimed in claim 1, in which $R^1$ is methyl, and n is an integer 1, 2 or 3.

3. The process for the preparation of a compound of the formula I as claimed in one or more of claims 1 and 2, in which $R^2$ denotes an amino acid residue which is protected with a urethane protective group, and $R^3$ denotes hydrogen.

4. The process for the preparation of a compound of the formula I as claimed in one or more of claims 1 to 3, in which the radicals $Y^1$, $Y^2$, $Y^3$, $Y^4$, $Y^5$, $Y^6$, $Y^7$, $Y^8$ and $Y^9$ represent methyl or methoxy, with, however, one of these radicals denoting $-O-(CH_2)_n-COOH$ and at least 4 of these radicals denoting hydrogen.

5. The process for the preparation of a compound of the formula I as claimed in one or more of claims 1 to 4, in which the radicals $Y^1$, $Y^3$, $Y^5$, $Y^7$ or $Y^8$ represent the radical $-O-(CH_2)_n-COOH$.

6. A process for the preparation of a peptide of the formula XI,

$$P-R^2NH-R^3 \quad (XI)$$

in which P represents a peptide residue composed of $q \leq p + 1$ $\alpha$-amino acids, $R^2$ denotes an amino acid residue, and $R^3$ is as defined in claim 1, by solid phase synthesis, which comprises coupling a compound of the formula I as claimed in one or more of claims 1 to 5, in which $R^2$ does not denote hydrogen, to a resin, cleaving off the protective group on the amino acid $R^2$, stepwise coupling on of q-p $\alpha$-amino acids which are, where appropriate, in the form of their activated derivatives and have been temporarily protected by amino protective groups which are base-labile or labile to weak acids, and, after the synthesis is complete, liberation of the peptide of the formula XI from the resin by treatment with a moderately strong acid, with temporarily introduced sidechain protective groups being cleaved off again at the same time or by suitable measures subsequent thereto.

7. A process for the preparation of a compound of the formula II,

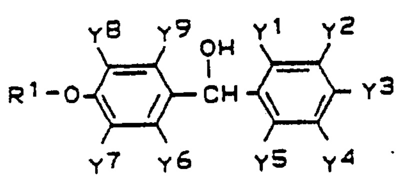

(II)

in which

$R^1$ denotes $(C_1-C_8)$-alkyl or optionally substituted $(C_6-C_{14})$-arryl,

$Y^1$,$Y^2$, $Y^3$, $Y^4$, $Y^5$, $Y^6$, $Y^7$, $Y^8$ and $Y^9$ denote hydrogen, $(C_1-C_4)$-alkyl, $(C_1-C_4)$-alkoxy or $-O-(CH_2)_n-COOH$, it being possible for the radicals to be identical or different but one radical being $-O-(CH_2)_n-COOH$, and

n denotes an integer from 1 to 6, which comprises reducing benzophenone derivatives of the formula IV

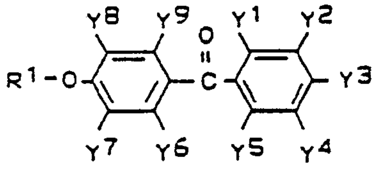

(IV)

in which $R^1$ , $Y^1$, $Y^2$, $Y^3$, $Y^4$, $Y^5$, $Y^6$, $Y^7$, $Y^8$ and $Y^9$ are as defined above.

## Revendications

### Revendications pour les Etats Contractants AT, BE, CH, DE, FR, GB, GR, IT, LI, LU, NL, SE

1. Composé de formule I

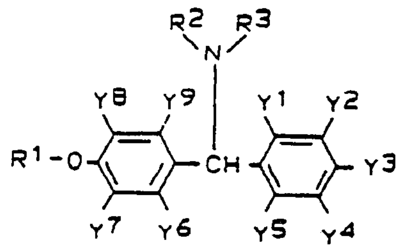

(I)

dans laquelle
$R^1$ représente un groupe alkyle en $C_1$-$C_8$ ou un groupe aryle en $C_6$-$C_{14}$ éventuellement substitué ;
$R^2$ représente un atome d'hydrogène ou un reste aminoacide qui est protégé par un groupe protecteur de fonction amino séparable en milieu faiblement acide ou basique ;
$R^3$ représente un atome d'hydrogène ou un groupe alkyle en $C_1$-$C_4$) ;
$Y^1$ , $Y^2$, $Y^3$, $Y^4$, $Y^5$, $Y^6$, $Y^7$, $Y^8$, $Y^9$ représentent des atomes d'hydrogène ou des groupes alkyle en $C_1$-$C_4$, alcoxy en $C_1$-$C_4$ ou $-O-(CH_2)_n-COOH$, les radicaux pouvant être identiques ou différents, un radical étant toutefois $-O-(CH_2)_n-COOH$, et
n est un nombre entier allant de 1 à 6.

2. Composé de formule I selon la revendication 1, dans lequel $R^1$ représente le groupe méthyle et n est un nombre entier égal à 1, 2 ou 3.

3. Composé de formule I selon une ou plusieurs des revendications 1 et 2, dans lequel $R^2$ représente un reste aminoacide qui est protégé par un groupe protecteur uréthanne et $R^3$ représente un atome d'hydrogène.

4. Composé de formule I selon une ou plusieurs des revendications 1 à 3, dans lequel les radicaux $Y^1$, $Y^2$, $Y^3$, $Y^4$, $Y^5$, $Y^6$, $Y^7$, $Y^8$ et $Y^9$ représentent des groupes méthyle ou méthoxy, un de ces radicaux étant toutefois $-O-(CH_2)_n-COOH$ et au moins 4 de ces radicaux représentant des atomes d'hydrogène.

5. Composé de formule I selon une ou plusieurs des revendications 1 à 4, dans lequel les radicaux $Y^1$, $Y^3$, $Y^5$, $Y^7$ ou $Y^8$ représentent le radical $-O-(CH_2)_n-COOH$.

6. Procédé pour la préparation d'un composé de formule I selon une ou plusieurs des revendications 1 à 5, caractérisé en ce que

a) on fait réagir un coposé de formule II

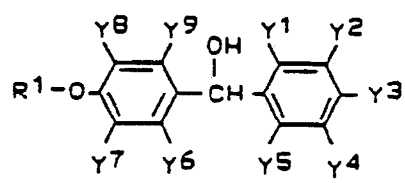

(II)

dans laquelle
$R^1$ représente un groupe alkyle en $C_1$-$C_8$ ou un groupe aryle en $C_6$-$C_{14}$ éventuellement substitué,
$Y^1$, $Y^2$, $Y^3$, $Y^4$, $Y^5$, $Y^6$, $Y^7$, $Y^8$, $Y^9$ représentent des atomes d'hydrogène ou des groupes alkyle en $C_1$-$C_4$, alcoxy en $C_1$-$C_4$ ou $-O-(CH_2)_n-COOH$, les radicaux pouvant être identiques ou différentes, un radical étant toutefois $-O-(CH_2)_n-COOH$, et
n représente un nombre entier allant de 1 à 6, avec un composé de formule III

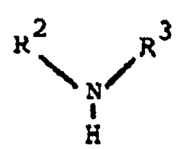

(III)

dans laquelle
$R^2$ représente un atome d'hydrogène ou un reste aminoacide qui est protégé par un groupe protecteur de fonction amino séparable en milieu faiblement acide ou basique, et
$R_3$ représente un atome d'hydrogène ou un groupe alkyle en $C_1$-$C_4$, ou
b) on fait réagir un composé de formule IV

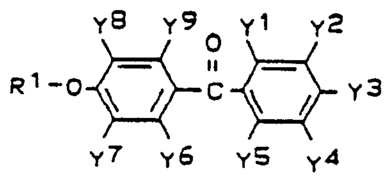

(IV)

avec de l'hydroxylamine, pour aboutir à un composé de formule V

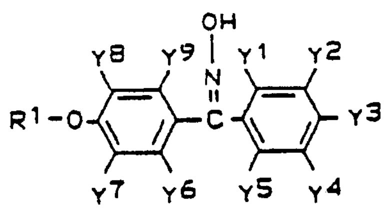

(V)

dans laquelle $R^1$, $Y^1$, $Y^2$, $Y^3$, $Y^4$, $Y^5$, $Y^6$, $Y^7$, $Y^8$ et $Y^9$ sont tels que définis en a), on réduit ensuite l'oxime en l'amine et éventuellement on la convertit en ses dérivés.

7. Utilisation d'un composé de formule I selon une ou plusieurs des revendications 1 à 5, dans lequel $R^2$ ne représente pas un atome d'hydrogène, dans la synthèse en phase solide de composés de formule XI

$$P-R^2-NH-R^3 \qquad (XI)$$

dans laquelle P représente un reste peptidique constitué de $q \leq p + 1$ α-aminoacides, $R^2$ représente un reste aminoacide et $R^3$ est tel que défini dans la revendication 1.

8. Procédé pour la préparation d'un peptide de formule XI dans lequel P, $R^2$ et $R^3$ sont tels que définis dans la revendication 7, par synthèse en phase solide, caractérisé en ce que l'on fixe à une résine un composé de formule I selon une ou plusieurs des revendications 1 à 5, dans lequel $R^2$ ne représente pas un atome d'hydrogène, on élimine le groupe protecteur de l'aminoacide $R^2$, on assemble graduellement q-p α-aminoacides éventuellement sous forme de leurs dérivés activés, et temporairement protégés par des groupes protecteurs de fonction amino labiles en présence de bases ou d'acides faibles, et une fois terminée la construction, on sépare d'avec la résine le peptide de formule XI par traitement par un acide de force moyenne, en séparant en même temps ou à la suite de cela, par des moyens appropriés, des groupes protecteurs de chaînes latérales, introduits temporairement.

9. Composé de formule II

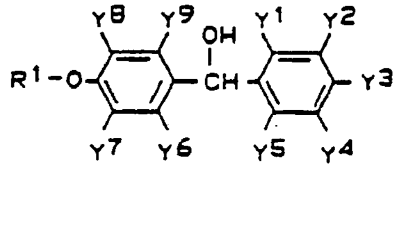

(II)

dans laquelle
$R^1$ représente un groupe alkyle en $C_1$-$C_8$ ou un groupe aryle en $C_6$-$C_{14}$ éventuellement substitué,
Y1, $Y^2$, $Y^3$, $Y^4$, $Y^5$, $Y^6$, $Y^7$, $Y^8$, $Y^9$ représentent des atomes d'hydrogène ou des groupes alkyle en $C_1$-$C_4$, alcoxy en $C_1$-$C_4$ ou $-O-(CH_2)_n-COOH$, les radicaux pouvant être identiques ou différents, un radical étant toutefois $-O-(CH_2)_n-COOH$, et
n est un nombre entier allant de 1 à 6.

**Pour l'Etat Contractant ES**

1. Procédé pour la préparation d'un composé de formule I

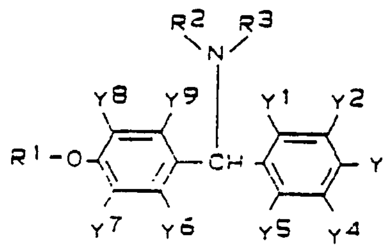

(I)

dans laquelle
$R^1$ représente un groupe alkyle en $C_1$-$C_8$ ou un groupe aryle en $C_6$-$C_{14}$ éventuellement substitué ;
$R^2$ représente un atome d'hydrogène ou un reste aminoacide qui est protégé par un groupe protecteur de fonction amino séparable en milieu faiblement acide ou basiques ;
$R^3$ représente un atome d'hydrogène ou un groupe alkyle en $Y^1$, $Y^2$, $Y^3$, $Y^4$, $Y^5$, $Y^6$, $Y^7$, $Y^8$, $Y^9$ représentent des atomes d'hydrogène ou des groupes alkyle en $C_1$-$C_4$, alcoxy en $C_1$-$C_4$ ou $-O-(CH_2)_n-COOH$, les radicaux pouvant être identiques ou différents, un radical étant toutefois $-O-(CH_2)_n-COOH$, et
n est un nombre entier allant de 1 à 6,
caractérisé en ce que

a) on fait réagir un composé de formule II

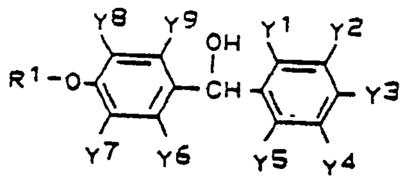

(II)

dans laquelle
$R^1$ représente un groupe alkyle en $C_1$-$C_8$ ou un groupe aryle en $C_6$-$C_{14}$ éventuellement substitué, $Y^1$, $Y^2$, $Y^3$, $Y^4$, $Y^5$, $Y^6$, $Y^7$, $Y^8$, $Y^9$ représentent des atomes d'hydrogène ou des groupes alkyle en $C_1$-$C_4$, alcoxy en $C_1$-$C_4$ ou $-O-(CH_2)_n-COOH$, les radicaux pouvant être identiques ou différents, un radical étant toutefois $-O-(CH_2)_n-COOH$, et
n représente un nombre entier allant de 1 à 6,
avec un composé de Formule III

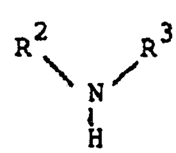

(III)

dans laquelle
$R^2$ représente un atome d'hydrogène ou un reste aminoacide qui est protégé par un groupe protecteur de fonction amino séparable en milieu faiblement acide ou basique, et
$R^3$ représente un atome d'hydrogène ou un groupe alkyle en $C_1$-$C_4$, ou

b) on fait réagir un composé de formule IV

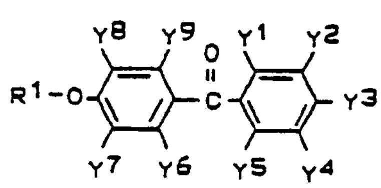

(IV).

avec de l'hydroxylamine, pour aboutir à un composé de formule V

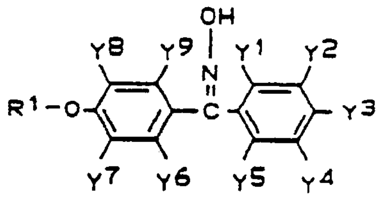

(V)

dans laquelle $R^1$, $Y^1$, $Y^2$, $Y^3$, $Y^4$, $Y^5$, $Y^6$, $Y^7$, $Y^8$ et $Y^9$ sont tels que définis en a),
n réduit ensuite l'oxime en l'amine et éventuellement on la convertit en ses dérivés.

2. Procédé pour la préparation d'un composé de formule I selon la revendication 1, dans lequel $R^1$ représente le groupe méthyle et n est un nombre entier égal à 1, 2 ou 3.

3. Procédé pour la préparation d'un composé de formule I selon une ou plusieurs des revendications 1 et 2, dans lequel $R^2$ représente un reste aminoacide qui est protégé par un groupe protecteur uréthane et $R^3$ représente un atome d'hydrogène.

4. Procédé pour la préparation d'un composé de formule I selon une ou plusieurs des revendications 1 à 3, dans lequel les radicaux $Y^1$, $Y^2$, $Y^3$, $Y^4$, $Y^5$, $Y^6$, $Y^7$, $Y^8$ et $Y^9$ représentent des groupes méthyle ou méthoxy, un de ces radicaux étant toutefois $-O-(CH_2)_n-COOH$ et au moins 4 de ces radicaux représentent des atomes d'hydrogène.

5. Procédé pour la préparation d'un composé de formule I selon une ou plusieurs des revendications 1 à 4, dans lequel les radicaux $Y^1$, $Y^3$, $Y^5$, $Y^7$ ou $Y^8$ représentent le radical $-O-(CH_2)n-COOH$.

6. Procédé pour la préparation d'un peptide de formule XI

$$P-R^2-NH-R^3 \quad (XI)$$

dans laquelle P représente un reste peptidique constitué de $q \leq p + 1$ α-aminoacides, $R^2$ représente un reste aminoacide et $R^3$ est tel que défini dans la revendication 1, par synthèse en phase solide, caractérisé en ce que l'on fixe à une résine un composé de formule I selon une ou plusieurs des revendications 1 à 5, dans lequel $R^2$ ne représente pas un atome d'hydrogène, on élimine le groupe protecteur de l'aminoacide $R^2$, on assemble graduellement q-p α-aminoacides éventuellement sous forme de leurs dérivés activés, et temporairement protégés par des groupes protecteurs de fonction amino labiles en présence de bases ou d'acides faibles, et une fois terminée la construction, on sépare d'avec la résine le peptide de formule XI par traitement par un acide de force moyenne, en séparant en même temps ou à la suite de cela par des moyens appropriés des groupes protecteurs de chaînes latérales, introduits temporairement.

7. Procédé pour la préparation d'un composé de formule II

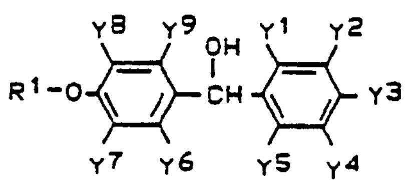

(II)

dans laquelle
$R^1$ représente un groupe alkyle en $C_1$-$C_8$ ou un groupe aryle en $C_6$-$C_{14}$ éventuellement substitué,
$Y^1$, $Y^2$, $Y^3$, $Y^4$, $Y^5$, $Y^6$, $Y^7$, $Y^8$, $Y^9$ représentent des atomes d'hydrogène ou des groupes alkyle en $C_1$-$C_4$, alcoxy en $C_1$ -$C_4$ ou $-O-(CH_2)_n-COOH$, les radicaux pouvant être identiques ou différents, un radical étant toutefois $-O-(CH_2)_n-COOH$, et
n est un nombre entier allant de 1 à 6, caractérisé en ce que l'on réduit des dérivés de benzophénone de formule IV

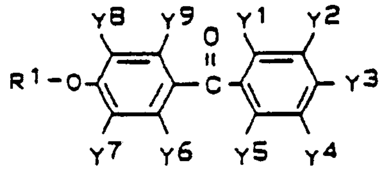

(IV)

dans laquelle $R^1$, $Y^1$ $Y^2$ $Y^3$ $Y^4$ $Y^5$ $Y^6$ $Y^7$ $Y^8$ et $Y^9$ sont tels que définis plus haut.